# EUROPEAN PATENT APPLICATION

(11) **EP 4 623 987 A1**
(43) Date of publication of application: **01.10.2025**
(21) Application number: 25165387.9
(22) Date of filing: 21.03.2025
(51) Int. Cl.: A61N 1/36, A61B 5/12, A61B 5/24, A61B 5/00

(54) **ELECTRODE ELECTRICAL STATUS-BASED DESIGNATION OF ELECTRODES OF AN IMPLANTABLE STIMULATOR FOR MEASURING AN EVOKED RESPONSE**

(30) Priority: 29.03.2024 US 202418622111
(71) Applicant: Advanced Bionics LLC, Valencia, CA 91355 (US)
(72) Inventor: Chen, Chen, CA Valencia, 91355 (US); Spahr, Anthony J., CA Newhall, 91321 (US); Koka, Kanthaiah, CA Valencia, 91355 (US)
(74) Representative: Schwan Schorer & Partner mbB

(57) **Abstract**

An exemplary electrical status-based determination of electrodes of an implantable stimulator for measuring an evoked response may include measuring electrical statuses for each electrode included in a plurality of electrodes of an implantable stimulator that is implanted within a recipient, designating, based on the electrical statuses, a first set of one or more electrodes included in the plurality of electrodes as stimulating electrodes to be used to deliver electrical stimulation to the recipient, and designating, based on the electrical statuses, a second set of one or more electrodes included in the plurality of electrodes as recording electrodes to be used to record one or more signals representative of one or more evoked responses that occur within the recipient in response to the electrical stimulation.

## Description

### BACKGROUND INFORMATION

An implantable stimulator may include or be coupled to one or more electrodes configured to be inserted within a recipient and used to apply electrical stimulation to the recipient at one or more locations. When stimulation is applied to the recipient of an implantable stimulator, the one or more electrodes may also be used to measure an evoked response that occurs within the recipient based on the stimulation.

As an example, the implantable stimulator may include a cochlear implant system (e.g., used to provide, restore, and/or improve the sense of hearing to recipients with severe or profound hearing loss). Conventional cochlear implant systems include various components configured to be implanted within a recipient (e.g., an electronics package, an antenna, and an electrode lead) and various components configured to be located external to the recipient (e.g., a sound processor, a battery, and a microphone). An electrode lead of a cochlear implant system may include an electrode array comprised of metal electrode contacts (e.g., platinum, titanium, etc.) configured to be inserted within a cochlea of a recipient and may be used to apply electrical stimulation to one or more intracochlear locations as well as measure an evoked response that occurs within the recipient based on the stimulation.

However, measuring an evoked response using an implantable stimulator and its electrodes is difficult due to challenging morphology and because the measured signals have a long acquisition window and a relatively small amplitude with competing artifacts and/or background noise.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 shows an illustrative configuration of an implantable stimulator system.
FIG. 2 shows another illustrative configuration of an implantable stimulator system that includes a cochlear implant.
FIG. 3 shows another illustrative configuration of an implantable stimulator system that includes a cochlear implant.
FIG. 4 shows another illustrative configuration of an implantable stimulator system.
FIG. 5 shows an illustrative method for determining electrodes of an implantable stimulator system for measuring an evoked response.
FIG. 6 shows another illustrative method for determining electrodes of an implantable stimulator system for measuring an evoked response.
FIG. 7 shows another illustrative method for determining electrodes of an implantable stimulator system for measuring an evoked response.
FIG. 8 shows another illustrative configuration of an implantable stimulator system.
FIG. 9 shows another illustrative configuration of an implantable stimulator system.
FIG. 10 shows an exemplary computing device.

### DETAILED DESCRIPTION

Systems and methods for electrode electrical status-based designation of electrodes of an implantable stimulator system for measuring an evoked response are described herein. For example, an implantable stimulator may include or be coupled to one or more electrodes configured to deliver electrical stimulation within a recipient and another one or more electrodes configured to record one or more signals representative of one or more evoked responses that occur within the recipient in response to the electrical stimulation. The one or more electrodes designated to deliver the electrical stimulation and/or the one or more electrodes designated to record the one or more signals are determined based on one or more electrical statuses associated with the one or more electrodes.

As used herein, an "evoked response" may include any type of neural response and/or cochlear response. Exemplary evoked responses include, but are not limited to, an electrocochleographic ("ECochG") potential (e.g., a cochlear microphonic potential, a compound action potential such as an auditory nerve response, a summating potential, etc.), a brainstem response (e.g., central auditory evoked potentials (CAEP) of a central auditory pathway of a recipient, an auditory brain stem response (ABR), a frequency following response (FFR), an auditory steady-state response (ASSR), etc.), an auditory evoked potential from auditory cortical areas (e.g., an auditory middle latency response (AMLR), an auditory 40 Hertz event-related response, an auditory late response (ALR), an auditory change complex (ACC), a cognitive or attentional response (P300), a mismatched negativity response (MMN), etc.), a stapedius reflex, and/or any other type of neural or physiological response that may occur within a recipient in response to application of stimulation to the recipient. Evoked responses may originate from neural tissues, hair cell to neural synapses, inner or outer hair cells, and/or other sources. Evoked responses such as those described herein may be used in any suitable manner. For example, evoked responses may be used to fit a hearing device (e.g., a cochlear implant, a hearing aid, etc.) to a recipient, to evaluate the recipient's hearing capability, and/or to monitor auditory brain maturation (e.g., pediatric brain development in relation to the effectiveness of a cochlear implant).

An illustrative system comprises a memory storing instructions and a processor configured to execute the instructions to perform a process. The process may include measuring impedances for each electrode included in a plurality of electrodes of an implantable stimulator that is implanted within a recipient, designating, based on the impedances, a first set of one or more electrodes included in the plurality of electrodes as stimulating electrodes to be used to deliver electrical stimulation to the recipient, and designating, based on the impedances, a second set of one or more electrodes included in the plurality of electrodes as recording electrodes to be used to record one or more signals representative of one or more evoked responses that occur within the recipient in response to the electrical stimulation.

An additional illustrative system comprises a cochlear implant including a plurality of electrodes positioned on an electrode lead at least partially within a cochlea of a recipient and a processing unit communicatively coupled to the cochlear implant. The processing unit may be configured to measure impedances for each electrode included in the plurality of electrodes, designate, based on the impedances, a first set of one or more electrodes included in the plurality of electrodes as stimulating electrodes to be used to deliver electrical stimulation to the recipient, and designate, based on the impedances, a second set of one or more electrodes included in the plurality of electrodes as recording electrodes to be used to record one or more signals representative of one or more evoked responses that occur within the recipient in response to the electrical stimulation.

An illustrative method comprises measuring impedances for each electrode included in a plurality of electrodes of an implantable stimulator that is implanted within a recipient, designating, based on the impedances, a first set of one or more electrodes included in the plurality of electrodes as stimulating electrodes to be used to deliver electrical stimulation to the recipient, and designating, based on the impedances, a second set of one or more electrodes included in the plurality of electrodes as recording electrodes to be used to record one or more signals representative of one or more evoked responses that occur within the recipient in response to the electrical stimulation.

The systems and methods described herein may provide various benefits to implantable stimulator recipients, such as cochlear implant recipients. For example, systems and methods such as those described herein may determine one or more electrodes of the implantable stimulator used to deliver the electrical stimulation and/or record the one or more signals based on one or more impedances associated with the one or more electrodes. By determining the one or more electrodes based on the one or more impedances associated with the one or more electrodes, competing artifacts (e.g., stimulation artifacts due to a residual charge from the application of the electrical stimulation itself) that interfere with measuring the evoked responses may be reduced and/or eliminated. Such a reduction and/or elimination of competing artifacts may further facilitate efficiently transmitting data indicative of evoked responses and automatically identifying key features (e.g., peaks, amplitudes, etc.) of the evoked responses in a clinically effective manner that minimizes the analytical burden of a clinician. Systems and methods such as those described herein may also assist a clinician in evaluating one or more conditions (e.g., a residual hearing status) of the recipient, and/or otherwise provide benefit to the recipient. For example, systems and methods such as those described herein may be beneficial for pediatric cochlear implant recipients and others that are unable to provide subjective feedback about their hearing.

Various embodiments will now be described in more detail with reference to the figures. The disclosed systems and methods may provide one or more of the benefits mentioned above and/or various additional and/or alternative benefits that will be made apparent herein.

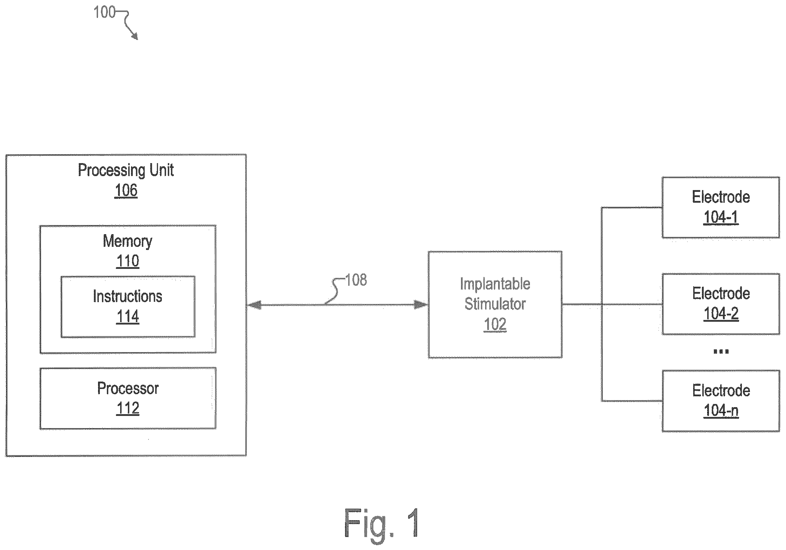

FIG. 1 shows an illustrative implantable stimulator system 100 configured to be used within a recipient for measuring one or more evoked responses within recipient due to electrical stimulation. As shown, implantable stimulator system 100 includes an implantable stimulator 102 ("stimulator 102"), a plurality of electrodes 104 (e.g., electrodes 104-1 through 104-n) coupled to stimulator 102, and a processing unit 106 configured to be communicatively coupled to stimulator 102 by way of a communication link 108.

Stimulator 102 may be implemented by any suitable type of device that may be implanted within the recipient and configured to apply electrical stimulation within the recipient by way of electrodes 104. Such electrical stimulation may be applied in any suitable manner using any suitable electrode or combination of electrodes. In some examples, stimulator 102 may be implemented by an implantable cochlear stimulator configured to apply electrical stimulation to an auditory pathway of the recipient (e.g., electrical stimulation may be applied by way of one or more electrodes 104 on an electrode lead at least partially inserted within a cochlea of a recipient). Additionally or alternatively, stimulator 102 may be implemented by a brainstem stimulator, an epilepsy stimulator (e.g., a Vagus nerve stimulator (VNS)), a spinal cord stimulator, a deep brain stimulator (DBS) and/or any other type of implantable stimulation device.

Electrodes 104 are formed of an electrically conductive material (e.g., a metal) and are electrically coupled (e.g., by way of one or more wires) to stimulator 102. To illustrate, stimulator 102 may include one or more current sources (not shown) electrically coupled with one or more electrodes 104 and that are configured to generate current delivered by way of the one or more electrodes 104 to one or more locations within the recipient. The electrical stimulation may be of any suitable type, such as monopolar stimulation, bipolar stimulation, etc. In some examples, stimulator 102 may include a plurality of independent current sources each associated with a channel defined by one or more of electrodes 104. In this manner, different current levels may be applied to multiple locations within the recipient simultaneously by way of multiple electrodes 104. Additionally, stimulator 102 may be configured to provide the current to the one or more electrodes 104 in accordance with varying stimulation parameters (e.g., amplitude, pulse width, pulse shape, frequency, polarity, duration, etc.).

Electrodes 104 may be configured to apply the electrical stimulation within the recipient at one or more stimulation sites within the recipient when electrodes 104 are stimulated by stimulator 102. The one or more stimulation sites may include one or more locations at which the one or more electrodes 104 are implanted within the recipient. For example, each electrode 104 may be implanted within the recipient such that each electrode is in operative contact (e.g., in physical contact with the recipient and/or within a distance of the recipient to sufficiently apply the electrical stimulation to the recipient) with the recipient, such as with tissue (e.g., cochlea, brain, spinal cord, etc.) of the recipient. A contact area between each electrode 104 and tissue of the recipient may be referred to as an electrode-tissue interface. Electrodes 104 may be implanted at different locations within a single area (e.g., cochlea) of the recipient and/or electrodes 104 may be implanted at different locations within multiple areas (e.g., cochlea, brain, brain stem, spinal cord, etc.) of the recipient.

Stimulator 102 may further include one or more return electrodes configured to provide a return path for the electrical stimulation applied by electrodes 104. For example, the one or more return electrodes may include a designated ground electrode, a housing (e.g., a case) of stimulator 102, and/or one or more non-stimulating electrodes 104 (e.g., one or more electrodes 104 that do not apply the electrical stimulation). In some examples, the one or more stimulation sites may further include the return path, such as tissue of the recipient between the one or more stimulating electrodes 104 (e.g., one or more electrodes 104 that apply the electrical stimulation) and the one or more return electrodes. The tissue included in the return path may be referred to as a tissue-tissue interface.

Stimulator 102 may additionally or alternatively be configured to generate, store, and/or transmit data. For example, stimulator 102 may use one or more electrodes 104 to record one or more signals (e.g., one or more voltages, impedances, stimulation artifacts, electrical statuses, evoked responses within the recipient, and/or other measurements) and transmit, by way of communication link 108, data representative of the one or more signals to processing unit 106. This data may be referred to as back telemetry data.

As an illustrative example, one or more electrodes 104 may record one or more signals representative of voltages detected by the one or more electrodes 104 in response to the electrical stimulation (e.g., at one or more electrode-tissue interfaces and/or tissue-tissue interfaces). In some examples, the one or more recording electrodes 104 may be configured to continuously record the one or more signals during an acquisition window. As used herein, "continuously record" may mean that implantable stimulator system 100 is in a recording mode during the entire acquisition window such that electrodes 104 are used to record evoked responses any time within the acquisition window. In certain examples, the "continuously recording" may include minimal gaps as long as the gaps do not shadow and/or void any evoked response characteristics of interest in a meaningful way. The acquisition window may have any suitable duration as may serve a particular implementation (e.g., such as between 0 seconds and 2 seconds, between 0 seconds and 1 second, between 0 milliseconds (ms) and 500 ms from applying the electrical stimulation, between 75 ms and 300 ms from applying the electrical stimulation, and/or between 100 ms and 200 ms from applying the electrical stimulation). The continuously recording of the data may be initiated prior to the stimulation being applied to the recipient and/or after the stimulation is applied to the recipient.

Processing unit 106 may be configured to interface with (e.g., control and/or receive data from) stimulator 102. For example, processing unit 106 may transmit commands (e.g., stimulation parameters and/or other types of operating parameters in the form of data words included in a forward telemetry sequence) to stimulator 102 by way of communication link 108. Processing unit 106 may additionally or alternatively provide operating power to stimulator 102 by transmitting one or more power signals to stimulator 102 by way of communication link 108. Processing unit 106 may additionally or alternatively receive data from stimulator 102 by way of communication link 108. Communication link 108 may be implemented by any suitable number of wired and/or wireless bidirectional and/or unidirectional links.

As shown, processing unit 106 includes a memory 110 and a processor 112 configured to be selectively and communicatively coupled to one another. In some examples, memory 110 and processor 112 may be distributed between multiple devices and/or multiple locations as may serve a particular implementation.

Memory 110 may be implemented by any suitable non-transitory computer-readable medium and/or non-transitory processor-readable medium, such as any combination of non-volatile storage media and/or volatile storage media. Exemplary non-volatile storage media include, but are not limited to, read-only memory, flash memory, a solid-state drive, a magnetic storage device (e.g., a hard drive), ferroelectric random-access memory ("RAM"), and an optical disc. Exemplary volatile storage media include, but are not limited to, RAM (e.g., dynamic RAM).

Memory 110 may maintain (e.g., store) executable data used by processor 112 to perform one or more of the operations described herein. For example, memory 110 may store instructions 114 that may be executed by processor 112 to perform any of the operations described herein. Instructions 114 may be implemented by any suitable application, program (e.g., sound processing program), software, code, and/or other executable data instance. Memory 110 may also maintain any data received, generated, managed, used, and/or transmitted by processor 112. Processor 112 may be configured to perform (e.g., execute instructions 114 stored in memory 110 to perform) various operations with respect to stimulator 102.

To illustrate, processor 112 may be configured to direct stimulator 102 to apply electrical stimulation to the recipient by way of one or more electrodes 104. In certain examples, the electrical stimulation may be applied to the recipient to elicit an evoked response. Processor 112 may be additionally or alternatively configured to receive and process data generated by stimulator 102. For example, processor 112 may receive data representative of one or more signals recorded by stimulator 102 using one or more electrodes 104 (e.g., non-stimulating electrodes).

In certain examples, the one or more electrodes 104 may be used in any suitable manner to measure an evoked response elicited within a recipient of stimulator 102 in response to the electrical stimulation. To illustrate, based on the one or more signals recorded by stimulator 102, processor 112 may be configured to process the one or more signals to determine one or more properties of an evoked response. The one or more properties of the evoked response may include any suitable property (e.g., one or more peaks, amplitudes of peaks, response latency, etc.) to identify and/or evaluate an evoked response. In some instances, processor 112 may determine whether a predefined evoked response parameter is satisfied. The predefined evoked response parameter may correspond to any suitable parameter that may indicate that the one or more signals include an evoked response. For example, the predefined evoked response parameter may correspond to a peak in the data that has an amplitude at or above a predefined amplitude.

Additionally or alternatively, after processing the one or more signals, processor 112 may be configured to output the one or more signals in any suitable format to be evaluated by a clinician. For example, the one or more signals may be presented in a chart (e.g., by way of a computing device) that includes any suitable information associated with the evoked response(s). In certain examples, the chart may include an auditory evoked potential waveform with one or more automatically identified peaks representing auditory evoked potentials of an auditory cortical area of the recipient.

Still other operations may be performed by processor 112 as may serve a particular implementation. For example, based on the one or more signals, processor 112 may use the data to perform one or more diagnostic operations with respect to stimulator 102 and/or the recipient. To illustrate, one or more operating parameters of stimulator 102 may be adjusted (e.g., one or more electrodes 104 used for stimulation, one or more electrodes 104 used for recording, one or more stimulation parameters, etc.). In the description provided herein, any references to operations performed by processing unit 106 and/or any implementation thereof may be understood to be performed by processor 112 based on instructions 114 stored in memory 110.

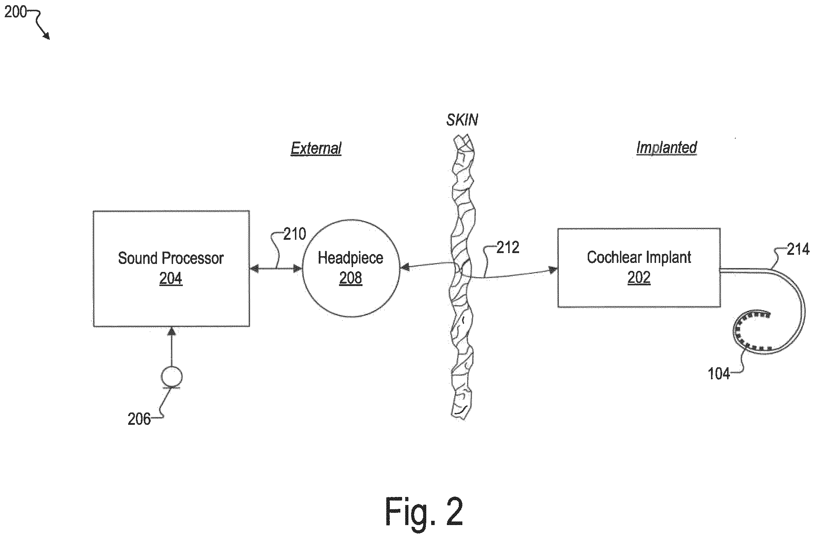

FIG. 2 shows an illustrative configuration 200 of implantable stimulator system 100 in which stimulator 102 is implemented by a cochlear implant 202 configured to be implanted internally within the recipient and processing unit 106 is implemented by a sound processor 204 configured to be located external to the recipient. In configuration 200, sound processor 204 is communicatively coupled to a microphone 206 and to a headpiece 208 that are both configured to be located external to the recipient.

Sound processor 204 may be implemented by any suitable device that may be worn or carried by the recipient. For example, sound processor 204 may be implemented by a behind-the-ear ("BTE") unit configured to be worn behind and/or on top of an ear of the recipient. Additionally or alternatively, sound processor 204 may be implemented by an off-the-ear unit (also referred to as a body worn device) configured to be worn or carried by the recipient away from the ear. Additionally or alternatively, at least a portion of sound processor 204 is implemented by circuitry within headpiece 208.

Microphone 206 is configured to detect one or more audio signals (e.g., that include speech and/or any other type of sound) in an environment of the recipient. Microphone 206 may be implemented in any suitable manner. For example, microphone 206 may be implemented by a microphone that is configured to be placed within the concha of the ear near the entrance to the ear canal, such as a T-MIC^{™} microphone from Advanced Bionics. Such a microphone may be held within the concha of the ear near the entrance of the ear canal during normal operation by a boom or stalk that is attached to an ear hook configured to be selectively attached to sound processor 204. Additionally or alternatively, microphone 206 may be implemented by one or more microphones in or on headpiece 208, one or more microphones in or on a housing of sound processor 204, one or more beam-forming microphones, and/or any other suitable microphone as may serve a particular implementation.

Headpiece 208 may be selectively and communicatively coupled to sound processor 204 by way of a communication link 210 (e.g., a cable or any other suitable wired or wireless communication link), which may be implemented in any suitable manner. Headpiece 208 may include an external antenna (e.g., a coil and/or one or more wireless communication components) configured to facilitate selective wireless coupling of sound processor 204 to cochlear implant 202. Headpiece 208 may additionally or alternatively be used to selectively and wirelessly couple any other external device to cochlear implant 202. To this end, headpiece 208 may be configured to be affixed to the recipient's head and positioned such that the external antenna housed within headpiece 208 is communicatively coupled to a corresponding implantable antenna (which may also be implemented by a coil and/or one or more wireless communication components) included within or otherwise connected to cochlear implant 202. In this manner, stimulation parameters and/or power signals may be wirelessly and transcutaneously transmitted between sound processor 204 and cochlear stimulator 102 by way of a wireless communication link 212.

In configuration 200, sound processor 204 may receive an audio signal detected by microphone 206 by receiving a signal (e.g., an electrical signal) representative of the audio signal from microphone 206. Sound processor 204 may additionally or alternatively receive the audio signal by way of any other suitable interface as described herein. Sound processor 204 may process the audio signal in any of the ways described herein and transmit, by way of headpiece 208, stimulation parameters to cochlear implant 202 to direct cochlear implant 202 to apply electrical stimulation representative of the audio signal to the recipient. Additionally or alternatively, sound processor 204 may be configured to transmit stimulation parameters to cochlear implant 202 to apply electrical stimulation using electrodes 104 to elicit an evoked response within the recipient.

In an alternative configuration, sound processor 204 may be implanted within the recipient instead of being located external to the recipient. In this alternative configuration, which may be referred to as a fully implantable configuration, sound processor 204 and cochlear implant 202 may be combined into a single device or implemented as separate devices configured to communicate one with another by way of a wired and/or wireless communication link. In a fully implantable configuration, headpiece 208 may not be included and microphone 206 may be implemented by one or more microphones implanted within the recipient, located within an ear canal of the recipient, and/or external to the recipient.

Cochlear implant 202 may include a cochlear stimulator configured to generate electrical stimulation in accordance with one or more stimulation parameters transmitted to cochlear implant 202 by sound processor 204 (e.g., representative of an audio signal processed by sound processor 204 and/or to elicit an evoked response within the recipient). Cochlear implant 202 may be further configured to apply the electrical stimulation to one or more stimulation sites (e.g., one or more intracochlear locations) within the recipient by way of one or more electrodes 104 on electrode lead 214. In some examples, cochlear implant 202 may include a plurality of independent current sources each associated with a channel defined by one or more of electrodes 104. In this manner, different stimulation current levels may be applied to multiple stimulation sites simultaneously by way of multiple electrodes 104.

Cochlear implant 202 may additionally or alternatively be configured to generate, store, and/or transmit data. For example, cochlear implant 202 may use one or more electrodes 104 to record one or more signals (e.g., one or more voltages, impedances, stimulation artifacts, electrical statuses, evoked responses within the recipient, and/or other measurements) and transmit, by way of communication link 212, data representative of the one or more signals to sound processor 204. The one or more of electrodes 104 electrically coupled to a cochlear implant 202 may be used in any suitable manner to measure an evoked response elicited within a recipient of cochlear implant 202 due to the electrical stimulation.

Electrode lead 214 may be implemented in any suitable manner. For example, a distal portion of electrode lead 214 may be pre-curved such that electrode lead 214 conforms with the helical shape of the cochlea after being implanted. Electrode lead 214 may alternatively be naturally straight or of any other suitable configuration. In some examples, electrode lead 214 includes a plurality of wires (e.g., within an outer sheath) that conductively couple electrodes 104 to one or more current sources within cochlear implant 202. For example, if there are n electrodes 104 on electrode lead 214 and n current sources within cochlear implant 202, there may be n separate wires within electrode lead 214 that are configured to conductively connect each electrode 104 to a different one of the n current sources. Exemplary values for n are 8, 12, 16, or any other suitable number.

Electrodes 104 are located on at least a distal portion of electrode lead 214. In this configuration, after the distal portion of electrode lead 214 is inserted into the cochlea, electrical stimulation may be applied by way of one or more of electrodes 104 to one or more intracochlear locations. One or more other electrodes (e.g., including a return electrode, not explicitly shown) may also be disposed on other parts of electrode lead 214 (e.g., on a proximal portion of electrode lead 214) to, for example, provide a return path for current applied by electrodes 104 and to remain external to the cochlea after the distal portion of electrode lead 214 is inserted into the cochlea. Additionally or alternatively, a housing of cochlear implant 202 may serve as a return electrode for current applied by electrodes 104.

Configuration 200 shown in FIG. 2 is a unilateral cochlear implant system (i.e., associated with only one ear of the recipient). Alternatively, a bilateral configuration of cochlear implant system may include separate implantable cochlear stimulators and electrode leads for each ear of the recipient. In the bilateral configuration, sound processor 204 may be implemented by a single processing unit configured to interface with both cochlear implants or by two separate processing units each configured to interface with a different one of the cochlear implants.

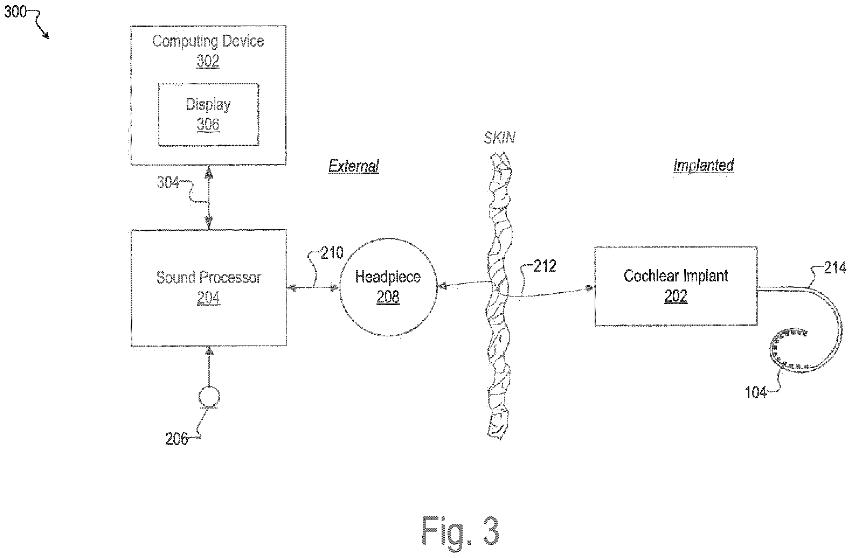

FIG. 3 shows another illustrative configuration 300 of implantable stimulator system 100 in which stimulator 102 is implemented by a cochlear implant 202 and processing unit 106 is implemented by a combination of sound processor 204 and a computing device 302 configured to communicatively couple to sound processor 204 by way of a communication link 304, which may be implemented by any suitable wired or wireless communication link.

Computing device 302 may be implemented by any suitable combination of hardware and software. To illustrate, computing device 302 may be implemented by a mobile device (e.g., a mobile phone, a laptop, a tablet computer, etc.), a desktop computer, and/or any other suitable computing device as may serve a particular implementation. As an example, computing device 302 may be implemented by a mobile device configured to execute an application (e.g., a "mobile app") that may be used by a user (e.g., the recipient, a clinician, and/or any other user) to control one or more settings of sound processor 204 and/or cochlear implant 202 and/or perform one or more operations (e.g., diagnostic operations) with respect to data generated by sound processor 204 and/or cochlear implant 202.

In some examples, computing device 302 may be configured to control an operation of cochlear implant 202 by transmitting one or more commands to cochlear implant 202 by way of sound processor 204. Likewise, computing device 302 may be configured to receive data generated by cochlear implant 202 by way of sound processor 204. Alternatively, computing device 302 may interface with (e.g., control and/or receive data from) cochlear implant 202 directly by way of a wireless communication link between computing device 302 and cochlear implant 202. In some implementations in which computing device 302 interfaces directly with cochlear implant 202, sound processor 204 may or may not be included in the cochlear implant system.

Computing device 302 is shown as having an integrated display 306. Display 306 may be implemented by a display screen, for example, and may be configured to display content generated by computing device 302. Additionally or alternatively, computing device 302 may be communicatively coupled to an external display device (not shown) configured to display the content generated by computing device 302.

In some examples, computing device 302 represents a fitting device configured to be selectively used (e.g., by a clinician) to fit sound processor 204 and/or cochlear implant 202 to the recipient. In these examples, computing device 302 may be configured to execute a fitting program configured to set one or more operating parameters of sound processor 204 and/or cochlear implant 202 to values that are optimized for the recipient. As such, in these examples, computing device 302 may not be considered to be part of the cochlear implant system. Instead, computing device 302 may be considered to be separate from the cochlear implant system such that computing device 302 may be selectively coupled to the cochlear implant system when it is desired to fit sound processor 204 and/or cochlear implant 202 to the recipient.

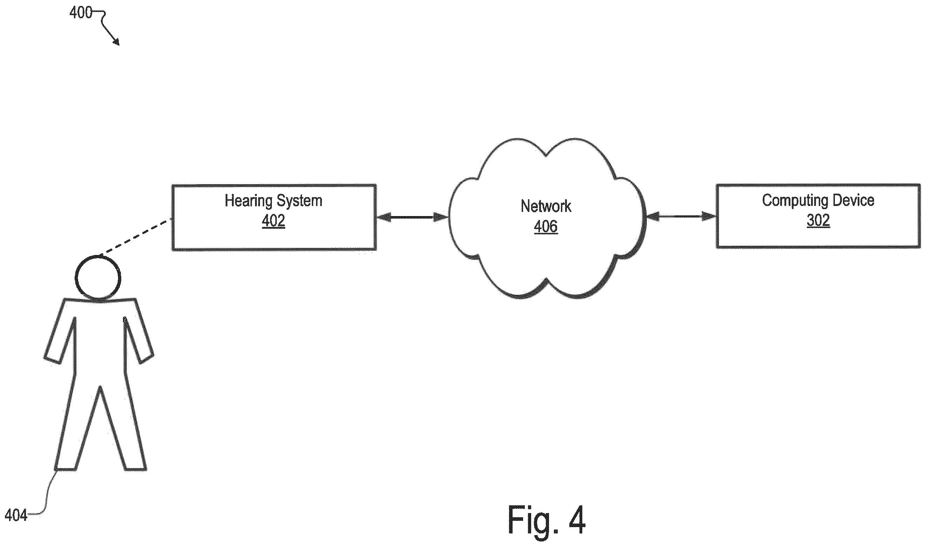

FIG. 4 shows another illustrative configuration 400 of implantable stimulator system 100 where a hearing system 402 of a recipient 404 is communicatively coupled to computing device 302 by way of a network 406. Hearing system 402 may be implemented by any suitable hearing device or combination of hearing devices as may serve a particular implementation.

As used herein, a "hearing device" may be implemented by any device or combination of devices configured to provide or enhance hearing to a user. For example, a hearing device may be implemented by a cochlear implant system such as described herein. In addition, a hearing device may include a hearing aid configured to amplify audio content to a recipient, a sound processor included in an implantable stimulator system configured to apply electrical stimulation to a recipient, an additional cochlear implant, or any other suitable hearing prosthesis. In some examples, a hearing device may be implemented by a behind-the-ear ("BTE") housing configured to be worn behind an ear of a user. In some examples, a hearing device may be implemented by an in-the-ear ("ITE") component configured to at least partially be inserted within an ear canal of a user. In some examples, a hearing device may include a combination of an ITE component, a BTE housing, and/or any other suitable component.

In certain examples, hearing devices such as those described herein may be implemented as part of an electro-acoustic stimulation ("EAS") system. Such an EAS system combines the functionality of a hearing aid and a cochlear implant system together in the same ear by providing acoustic stimulation representative of low frequency audio content and electrical stimulation representative of high frequency content.

In certain examples, hearing devices such as those described herein may be implemented as part of a binaural hearing system. Such a binaural hearing system may include a first hearing device associated with a first ear of a recipient and a second hearing device associated with a second ear of a recipient. In such examples, the hearing devices may each be implemented by any type of hearing device configured to provide or enhance hearing to a user of a binaural hearing system. In some examples, the hearing devices in a binaural system may be of the same type. For example, the hearing devices may each be cochlear implant devices. In certain alternative examples, the hearing devices may be of a different type. For example, a first hearing device may be a hearing aid and a second hearing device may be a sound processor included in a cochlear implant system.

Network 406 may include, but is not limited to, one or more wireless networks (Wi-Fi networks), wireless communication networks, mobile telephone networks (e.g., cellular telephone networks), mobile phone data networks, broadband networks, narrowband networks, the Internet, local area networks, wide area networks, and any other networks capable of carrying data and/or communications signals between hearing system 402 and computing device 302. In certain examples, network 406 may be implemented by a Bluetooth protocol (e.g., Bluetooth Classic, Bluetooth Low Energy ("LE"), etc.) and/or any other suitable communication protocol to facilitate communications between hearing system 402 and computing device 302. In certain examples, network 406 may include a back telemetry channel that communicatively couples a cochlear implant of hearing system 402 to computing device 302 and/or any other suitable device/system (e.g., external components of a cochlear implant system). Communications between hearing system 402, computing device 302, and any other device/system may be transported using any one of the above-listed networks, or any combination or sub-combination of the above-listed networks. Additionally or alternatively, computing device 302 may be directly coupled to hearing system 402, e.g., by way of a wired connection.

In instances when implantable stimulator system 100 is configured to measure an evoked response within the recipient, implantable stimulator system 100 may detect competing artifacts that may interfere with measuring the evoked response. For example, electrodes 104 implanted within the recipient and used for both electrical stimulation and recording may share the same circuitry and/or environment, which may cause interactions between the stimulating electrodes 104 and the recording electrodes 104 and/or competing artifacts. Such competing artifacts may include stimulation artifacts due to a residual charge from the application of the electrical stimulation itself. To illustrate, the stimulation artifacts may result from charge build-up (e.g., residual voltages at one or more electrodes 104, one or more electrode-tissue interfaces, and/or one or more tissue-tissue interfaces) due to applying the electrical stimulation using one or more stimulating electrodes 104 of implantable stimulator system 100. Accordingly, the one or more signals recorded by the one or more recording electrodes 104 of implantable stimulator system 100 may include one or more signals associated with the charge build-up (e.g., by detecting the residual voltages) in addition to or instead of the evoked response of the recipient. These stimulation artifacts may interfere with measuring the evoked response within the recipient such as until the simulation artifacts dissipate. One or more properties (e.g., a magnitude, a frequency, a duration, etc.) of the stimulation artifacts may be indicative of one or more of impedances of electrodes 104, a quality of one or more electrode-tissue interfaces, a quality of one or more tissue-tissue interfaces, or one or more stimulation parameters associated with the electrical stimulation.

As illustrative examples, signals representative of voltages detected by one or more electrodes (e.g., electrodes 104) of an implantable stimulator system (e.g., implantable stimulator system 100) may be received in response to one or more other electrodes (e.g., electrodes 104) of the implantable stimulator system applying electrical stimulation within a recipient as a function of time at various saline concentration levels. For example, each saline concentration level may correspond to an impedance of the electrodes (e.g., the one or more electrodes applying the electrical stimulation). The voltages may be measured during an acquisition window between 0 ms and 500 ms from the application of the electrical stimulation. Such voltages may be indicative of impedances of the electrodes, stimulation artifacts due to the electrical stimulation, and/or evoked responses within the recipient elicited by the electrical stimulation. To illustrate, at least an initial peak of each signal may be indicative of impedances and/or stimulation artifacts due to the electrical stimulation.

For a signal representative of voltages in response to electrical stimulation as a function of time at a saline concentration level that corresponds to an impedance of about 20,000 ohms, an initial peak of the signal may include a large amplitude that exceeds predetermined voltage values (e.g., +/- 20 microvolts (µV)) and have a duration of most of the acquisition window (e.g., 350 ms). The signal may further include a noise floor of about 16 µV, a peak-to-peak noise of about 200 µV, and a signal-to-noise ratio of about 6.2. This signal may be indicative of a large stimulation artifact associated with the implantable stimulator system that may highly interfere with measuring an evoked response elicited by the electrical stimulation.

As another example, a signal representative of voltages in response to electrical stimulation as a function of time at a saline concentration level that corresponds to an impedance of about 15,000 ohms may include an initial peak having an amplitude that exceeds the predetermined voltage values (e.g., +/- 20 microvolts (µV)) and a duration of about 125 ms. The signal may further include a noise floor of about 5.6 µV, a peak-to-peak noise of about 40 µV, and a signal-to-noise ratio of about 3.6. This signal may be indicative of a pronounced stimulation artifact associated with the implantable stimulator system that may interfere with measuring an evoked response elicited by the electrical stimulation (e.g., during the initial peak of the signal).

A signal representative of voltages in response to electrical stimulation as a function of time at a saline concentration level that corresponds to an impedance of about 7,000 ohms may include an initial peak having a reduced amplitude that stays within the predetermined voltage values (e.g., +/- 20 microvolts (µV)) and a decreased duration of about 25 ms. The signal may further include a noise floor of about 2.8 µV, a peak-to-peak noise of about 19 µV, and a signal-to-noise ratio of about 3.3. This signal may be indicative of a noticeable stimulation artifact associated with the implantable stimulator system that may interfere with measuring an evoked response elicited by the electrical stimulation (e.g., during the initial peak of the signal).

A signal representative of voltages in response to electrical stimulation as a function of time at a saline concentration level that corresponds to an impedance of about 3,000 ohms may include an initial peak having a minimal amplitude and duration. The signal may further include a noise floor of about 4.9 µV, a peak-to-peak noise of about 17 µV, and a signal-to-noise ratio of about 1.7. This signal may be indicative of a minimal stimulation artifact associated with the implantable stimulator system that may minimally interfere with measuring an evoked response elicited by the electrical stimulation.

Accordingly, the examples described above illustrate that the stimulation artifacts associated with the implantable stimulator system correspond to impedances of the electrodes such that the stimulation artifacts decrease with reduced impedances. Due to the correlation between the impedances and the stimulation artifacts, illustrative methods are described below for using the impedances to designate a first group of one or more electrodes of an implantable stimulator system as stimulating electrodes that are to be used to deliver electrical stimulation to the recipient and a second group of one or more electrodes as recording electrodes that are to be used to record one or more signals elicited in response to the electrical stimulation. This may reduce and/or eliminate stimulation artifacts that may interfere with measuring one or more evoked responses that occur within the recipient in response to the electrical stimulation. Because impedances and/or stimulation artifacts associated with the electrodes may vary over time (e.g., due to movement of the electrodes within the recipient, fold over of an electrode lead, changes between contact of the electrodes and tissue of the recipient, environment changes (e.g., tissue growth), etc.), the electrodes may be determined prior to measuring the one or more evoked responses, such as when the implantable stimulator is implanted, prior to executing a fitting program, and/or periodically (e.g., hourly, daily, weekly, monthly, etc.).

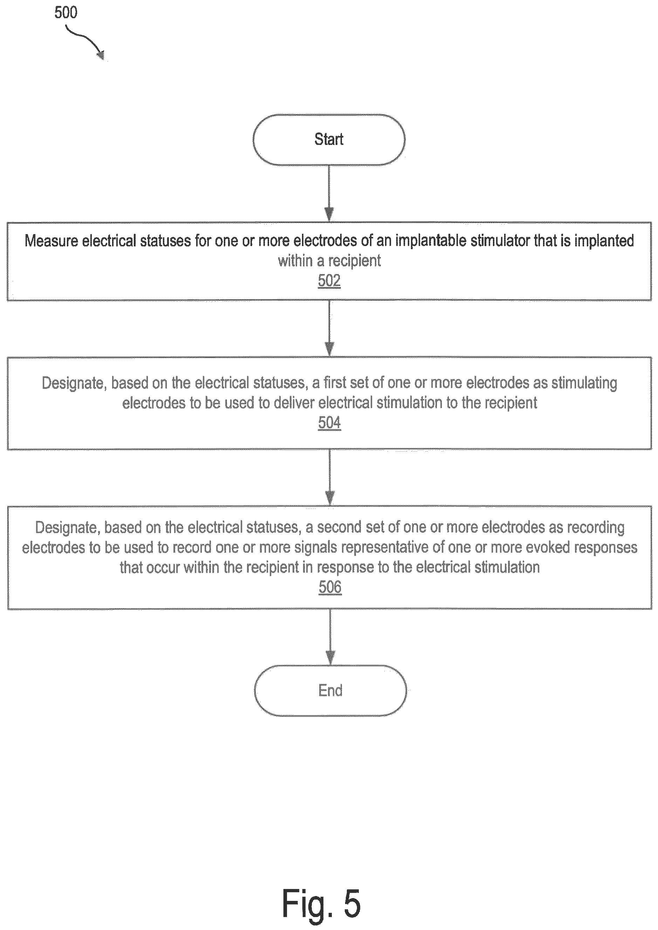

For example, FIG. 5 depicts an illustrative method 500 for determining electrodes (e.g., electrodes 104) of an implantable stimulator (e.g., stimulator 102) included in an implantable stimulator system (e.g., implantable stimulator system 100) configured to measure one or more evoked responses within a recipient in response to electrical stimulation. While FIG. 5 illustrates exemplary operations according to one embodiment, other embodiments may omit, add to, reorder, and/or modify any of the operations shown in FIG. 5. Moreover, each of the operations depicted in Fig. 5 may be performed in any of the ways described herein. One or more of the operations depicted in Fig. 5 may be performed by a processing unit (e.g., processing unit 106) of the implantable stimulator system.

As shown, method 500 may include, at operation 502, measuring electrical statuses (e.g., voltages, stimulation artifacts, impedances such as access impedances and/or polarization impedances, etc.) for one or more electrodes included in a plurality of electrodes of an implantable stimulator that is implanted within a recipient. For example, the measuring the electrical statuses may include measuring impedances for the one or more electrodes such as by sequentially applying current by way of each electrode and/or various combinations of electrodes included in the plurality of electrodes. Such applied current may produce a voltage (e.g., between each stimulated electrode and a return electrode) in response to the current. Accordingly, the measuring the impedances may further include measuring voltages associated with each applied current, such as by using one or more of the non-stimulated electrodes of the implantable stimulator to record one or more signals representative of the voltages (e.g., when each current is applied). The measured voltages may be representative of voltages at one or more electrode-tissue interfaces and/or one or more tissue-tissue interfaces (e.g., along the return path). The measuring the impedances may further include determining, based on the voltages, impedances for each electrode and/or combinations of electrodes. For example, the impedances for each electrode and/or combination of electrodes that applied current may be derived based on the measured voltages relative to the current applied by the corresponding electrode and/or combination of electrodes. Each of the impedance measurements may include a capacitive component and a polarization component. In some implementations, electric field imaging (EFI) may be used to capture a whole trans-impedance matrix (TIM) for the plurality of electrodes included in the implantable stimulator.

The measuring the electrical statuses may additionally or alternatively include measuring stimulation artifacts associated with the for the one or more electrodes. For example, various combinations of electrodes included in the plurality of electrodes may sequentially apply electrical stimulation and record one or more signals representative of stimulation artifacts in response to the electrical stimulation (e.g., by detecting voltages at the one or more electrodes).

Method 500 may further include, at operation 504, designating, based on the electrical statuses, a first set of one or more electrodes included in the plurality of electrodes as stimulating electrodes to be used to deliver electrical stimulation to the recipient. In some implementations, the first set of one or more electrodes may be designated based on one or more minimum impedances and/or stimulation artifacts associated with the one or more electrodes. For example, an electrode having a minimum impedance and/or a group of electrodes having a collective minimum impedance may be designated as the first set of electrodes. A minimum impedance may refer to an electrode and/or group of electrodes having an impedance that is lower than the impedance of another electrode and/or group of electrodes. Additionally or alternatively, a minimum impedance may refer to an electrode and/or group of electrodes having the lowest impedance relative to the impedances of each of the other electrodes and/or groups of electrodes. Because impedances associated with the electrodes of the implantable stimulator correspond to stimulation artifacts associated with the implantable stimulator, designating the first set of one or more electrodes may reduce and/or eliminate such stimulation artifacts. Additionally or alternatively, one or more electrodes included in a combination of electrodes associated with minimum stimulation artifacts may be designated as the first set of electrodes.

Method 500 may further include, at operation 506, designating, based on the electrical statuses, a second set of one or more electrodes included in the plurality of electrodes as recording electrodes to be used to record one or more signals representative of one or more evoked responses that occur within the recipient in response to the electrical stimulation. In some implementations, the second set of one or more electrodes may also be designated based on one or more minimum impedances and/or stimulation artifacts associated with the one or more electrodes. For example, an electrode having a minimum impedance and/or a group of electrodes having a collective minimum impedance may be designated as the second set of electrodes. Accordingly, designating the second set of one or more electrodes based on one or more minimum impedances may reduce and/or eliminate stimulation artifacts associated with the implantable stimulator. Additionally or alternatively, one or more electrodes included in a combination of electrodes associated with minimum stimulation artifacts may be designated as the second set of electrodes.

The one or more electrodes included in the second set of one or more electrodes differ from the one or more electrodes included in the first set of one or more electrodes. To illustrate, the first set of one or more electrodes may be designated prior to the second set of one or more electrodes such that the second set of one or more electrodes may be based on one or more minimum impedances of one or more remaining electrodes that were not included in the first set of one or more electrodes. Alternatively, the second set of one or more electrodes may be designated prior to the first set of one or more electrodes such that the first set of one or more electrodes may be based on one or more minimum impedances of one or more remaining electrodes that were not included in the second set of one or more electrodes. Moreover, a combination of electrodes associated with minimum impedances may be separated into the first set of one or more electrodes and the second set of one or more electrodes.

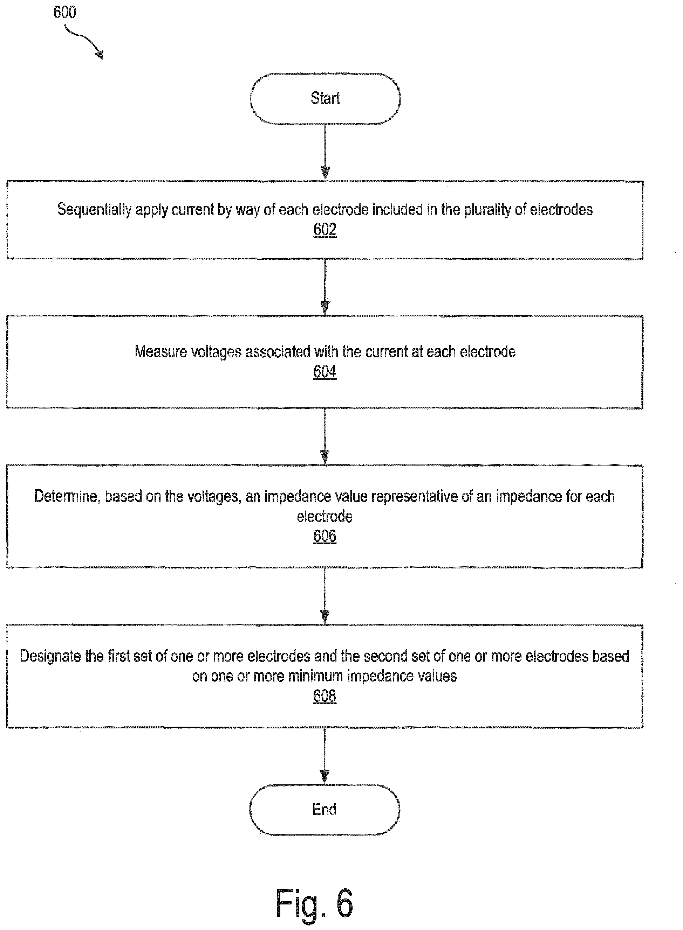

FIG. 6 depicts another illustrative method 600 for determining electrodes (e.g., electrodes 104) of an implantable stimulator (e.g., stimulator 102) included in an implantable stimulator system (e.g., implantable stimulator system 100) configured to measure one or more evoked responses within a recipient in response to electrical stimulation. While FIG. 6 illustrates exemplary operations according to one embodiment, other embodiments may omit, add to, reorder, and/or modify any of the operations shown in FIG. 6. Moreover, each of the operations depicted in Fig. 6 may be performed in any of the ways described herein. One or more of the operations depicted in Fig. 6 may be performed by a processing unit (e.g., processing unit 106) of the implantable stimulator system.

As shown, method 600 may include, at operation 602, sequentially applying current by way of each electrode included in the plurality of electrodes. For example, a first electrode may be stimulated with current (e.g., by stimulator 102) prior to a second electrode being stimulated with current, and so on (e.g., until each of the electrodes included in the plurality of electrodes have been stimulated with current). In some examples, a sufficient amount of time may be provided between each application of current, such as an acquisition window for measuring voltages in response to the corresponding application of current.

Accordingly, method 600 may further include, at operation 604, measuring voltages associated with the current applied by each electrode. For example, one or more of the non-stimulated electrodes may be used to record one or more signals representative of voltages when the current is applied to each electrode (e.g., during the acquisition window). The measured voltages may be representative of voltages at one or more electrode-tissue interfaces and/or one or more tissue-tissue interfaces (e.g., along the return path).

Method 600 may further include, at operation 606, determining, based on the voltages, an impedance value representative of an impedance for each electrode. For example, the impedance value for each electrode may be determined based on the voltages measured in response to the current applied by the electrode. The impedance value may be represented by any suitable metric, such as a discrete value (e.g., a value, a percentage, a level, a range, etc.) representative of an impedance for the corresponding electrode. The impedance values may include a capacitive component and a polarization component of the impedance.

Method 600 may further include, at operation 608, designating the first set of one or more electrodes and the second set of one or more electrodes based on one or more minimum impedance values. For example, a minimum impedance value may include one or more of an impedance value that is below an impedance threshold (e.g., at which stimulation artifacts are produced and/or increased), an impedance value that is lower than another impedance value, or an impedance value that is less than each of the other impedance values. An electrode associated with a minimum impedance value may be designated within the first set of electrodes or the second set of electrodes. In some examples, a group of electrodes that are each associated with a minimum impedance value may be designated as the first set of electrodes or the second set of electrodes.

Additionally or alternatively, an impedance value for each electrode included in a group of electrodes may be combined to form a combined impedance value representative of impedances for the group of electrodes such that the first set of one or more electrodes and/or the second set of one or more electrodes may be designated based on the combined impedance value. To illustrate, a group of electrodes associated with a minimum (e.g., lower) combined impedance value relative to another group of electrodes may be designated as the first set of electrodes or the second set of electrodes. Additionally or alternatively, the group of electrodes associated with the minimum combined impedance value may be separated into the first set of one or more electrodes and the second set of one or more electrodes. Such minimum impedance values may correspond to reduced and/or eliminated stimulation artifacts such that designating the first and second sets of electrodes may reduce and/or eliminate stimulation artifacts prior to measuring an evoked response within the recipient. Still other suitable methods for designating the first and second sets of electrodes may be used.

For example, the designating the first set of one or more electrodes and/or the second set of one or more electrodes may be based on the second set of one or more electrodes being spaced a minimum distance away from the first set of one or more electrodes. To illustrate, stimulation artifacts associated with the implantable stimulator may increase when the distance between the stimulating electrodes and the recording electrodes decreases. Accordingly, one or more electrodes included in the second set of one or more electrodes may be spaced a minimum distance away from the one or more electrodes included in the first set of one or more electrodes such as to reduce the stimulation artifacts. As an illustrative example, for electrodes located on an electrode lead of a cochlear implant, the second set of one or more electrodes may be spaced a minimum distance away from the first set of one or more electrodes on the electrode lead (e.g., the first set of one or more electrodes may be positioned toward a distal end of the electrode lead away from the cochlear implant and the second set of one or more electrodes may be positioned toward a proximal end of the electrode lead toward the cochlear implant, or vice versa).

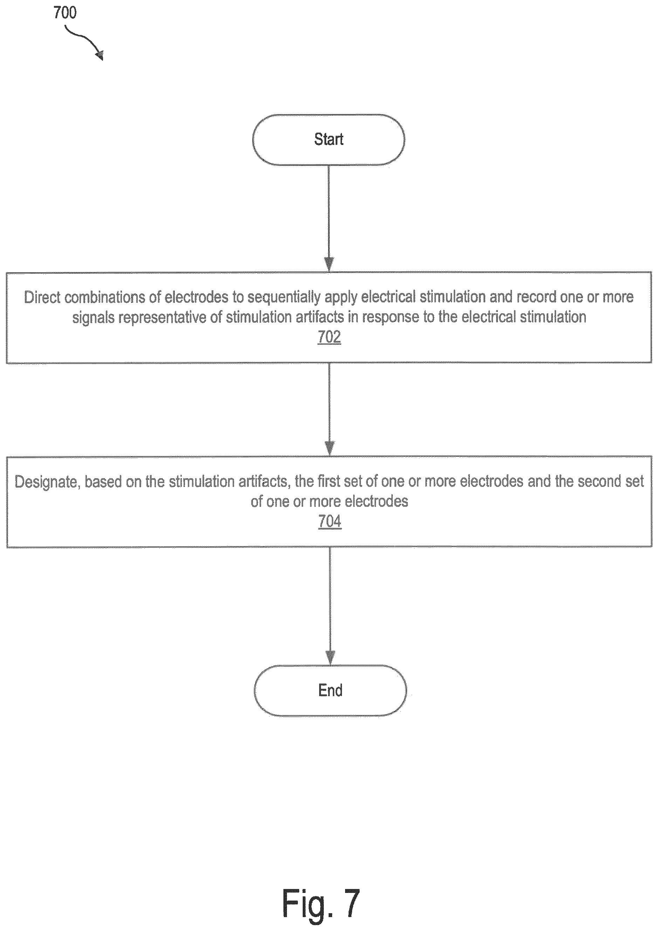

Additionally or alternatively, the designating the first and second sets of electrodes may be based measuring stimulation artifacts for various combinations of stimulating and recording electrodes. For example, FIG. 7 depicts an illustrative method 700 for determining electrodes (e.g., electrodes 104) of an implantable stimulator (e.g., stimulator 102) included in an implantable stimulator system (e.g., implantable stimulator system 100) based on stimulation artifacts. While FIG. 7 illustrates exemplary operations according to one embodiment, other embodiments may omit, add to, reorder, and/or modify any of the operations shown in FIG. 7. Moreover, each of the operations depicted in Fig. 7 may be performed in any of the ways described herein. One or more of the operations depicted in Fig. 7 may be performed by a processing unit (e.g., processing unit 106) of the implantable stimulator system.

As shown, method 700 includes, at operation 702, directing combinations of electrodes to sequentially apply electrical stimulation and record one or more signals representative of stimulation artifacts in response to the electrical stimulation. For example, one or more electrodes included in a first combination of electrodes may be stimulated with current (e.g., by stimulator 102) and one or more other electrodes included in the first combination of electrodes may record the one or more signals representative of the stimulation artifacts by detecting voltages at the one or more electrodes. After the recording acquisition window of the first combination of electrodes, a second combination of electrodes may apply current and record the one or more signals, and so on until each desired combination of electrodes included in the plurality of electrodes have been used to measure the stimulation artifacts associated with the combination of electrodes.

In some examples, the electrical stimulation may be applied below a stimulation threshold at which one or more evoked responses are evoked within the recipient. This may allow the stimulation artifacts to be identified without interference from the one or more evoked responses for reducing and/or eliminating the stimulation artifacts. Still other suitable methods may be used to apply the electrical stimulation by the combinations of electrodes. Additionally or alternatively, the electrical stimulation may be applied by the combinations of electrodes at increasing electrical stimulation levels and/or above the stimulation threshold to elicit one or more evoked responses within the recipient in addition to the stimulation artifacts. Additionally or alternatively, the electrical stimulation may be applied by the combinations of electrodes with varying stimulation parameters, such as at varying amplitudes, pulse widths, pulse shapes, frequencies, polarities, durations, etc. This may generate varying stimulation artifacts within the recipient such that one or more of the stimulation parameters may be selected for reducing and/or eliminating the stimulation artifacts. As an illustrative example, the electrical stimulation may be applied by the combinations of electrodes using varying asymmetric pulses of electrical stimulation. Additionally or alternatively, the electrical stimulation may include a post-pulse train electrical stimulus, such as a biphasic pulse train having multiple pulses that may be followed by one or more pseudo-monophasic pulses (e.g., to reduce or cancel residual charge). One or more characteristics (e.g., an amplitude, a sign, a width, etc.) of the final pseudo-monophasic pulse may be manipulated based on the post-pulse train electrical stimulus and/or stimulation artifacts.

Method 700 further includes, at operation 704, designating, based on the stimulation artifacts, the first set of one or more electrodes and the second set of one or more electrodes (e.g., to reduce the stimulation artifacts). For example, the first set of one or more electrodes and the second set of one or more electrodes may be designated based on one or more combinations of electrodes associated with minimum stimulation artifacts (e.g., stimulation artifacts lower than a threshold, stimulation artifacts lower than another combination of electrodes, stimulation artifacts lower than each of the other combinations of electrodes, etc.). For the combinations of electrodes determined to have minimum stimulation artifacts, the stimulation electrodes of the combinations may be designated as the first set of one or more electrodes and the recording electrodes of the combinations may be designated as the second set of one or more electrodes.

In some instances, a single combination of one or more stimulating electrodes and recording electrodes may be separated into the first set of one or more electrodes and the second set of one or more electrodes, respectively. Alternatively, multiple combinations of one or more stimulating and recording electrodes may be designated as the first and second sets of electrodes. For example, the sets of electrodes may be designated based on one or more combinations of electrodes having stimulation artifacts that may cancel and/or reduce the stimulation artifacts associated with another one or more combinations of electrodes. To illustrate, a first combination of stimulating and recording electrodes may produce stimulation artifacts having voltages in a positive direction and a second combination of stimulating and recording electrodes may produce stimulation artifacts having voltages in a negative direction such that the stimulation artifacts of the second combination of electrodes may cancel and/or reduce the stimulation artifacts of the first combination of electrodes. Accordingly, the stimulating electrodes of the first and second combinations may be designated as the first set of one or more electrodes and the recording electrodes of the first and second combinations may be designated as the second set of one or more electrodes to provide an overall reduction of stimulation artifacts. In some implementations, one or more electrodes included in the first set may be configured to apply electrical stimulation with stimulation parameters that differ than the electrical stimulation applied by another electrode included in the first set such as to reduce the stimulation parameters.

In some implementations, various combinations of stimulating and recording electrodes used to measure stimulation artifacts may be used to derive one or more characteristics associated with the stimulation artifacts and/or electrodes. For example, the one or more characteristics may include one or more of stimulation artifacts associated with an individual electrode included in the combinations of electrodes, stimulation artifacts associated with a pair and/or group of electrodes included in the combinations of electrodes, stimulation electrodes included in the combinations of electrodes, recording electrodes included in the combinations of electrodes, a return electrode, a return path, or one or more stimulation parameters. In some examples, the one or more characteristics may be derived automatically (e.g., using machine learning algorithms) by the implantable stimulator system based on the measured stimulation artifacts. To illustrate, one or more properties of the stimulation artifacts may include any suitable property (e.g., one or more peaks, amplitudes of peaks, response latency, etc.) to identify and/or evaluate the one or more characteristics. Such characteristics may further be used to designate the first and second sets of electrodes to reduce and/or eliminate the stimulation artifacts.

In some implementations, the stimulation artifacts for the various combinations of electrodes may be measured based on varying amplifier parameters associated with an amplifier of the implantable stimulator system. The amplifier parameters may include one or more of a connection of the amplifier, a disconnection of the amplifier, designated discharge resistors of the amplifier, designated shunt resistors of the amplifier, or designated discharge capacitors of the amplifier. Such amplifier parameters may be determined based on measured stimulation artifacts such as to reduce and/or eliminate stimulation artifacts.

As illustrative examples, signals including stimulation artifacts associated with an implantable stimulator system (e.g., implantable stimulator system 100) as a function of time may be received using various combinations of stimulating and recording electrodes. The signals may be recorded during an acquisition window between 0 ms and 500 ms from the application of the electrical stimulation. Such voltages may be indicative of impedances of the electrodes, stimulation artifacts due to the electrical stimulation, and/or evoked responses within the recipient elicited by the electrical stimulation. To illustrate, at least an initial peak of each signal may be indicative of impedances and/or stimulation artifacts due to the electrical stimulation.

For example, a signal representative of voltages in response to electrical stimulation as a function of time using a first combination of electrodes including a single stimulating electrode and a single recording electrode may have an initial peak that exceeds the predetermined voltage values (e.g., +/- 20 µV) and has a duration of about 125 ms. The signal may further include a noise floor of about 1.8 µV, a peak-to-peak noise of about 25 µV, and a signal-to-noise ratio of about 6.9. This signal may be indicative of a noticeable stimulation artifacts associated with the implantable stimulator system that may interfere with measuring an evoked response elicited by the electrical stimulation.

A signal representative of voltages in response to electrical stimulation as a function of time using a second combination of electrodes including a single stimulating electrode and multiple recording electrodes may have an initial peak that stays within the predetermined voltage values (e.g., +/- 20 µV) and has a duration of about 100 ms. The signal may further include a noise floor of about 2.1 µV, a peak-to-peak noise of about 8.2 µV, and a signal-to-noise ratio of about 1.9. This signal may be indicative of stimulation artifacts associated with the implantable stimulator system that are less than the stimulation artifacts associated with the first combination of electrodes, but that may still interfere with measuring an evoked response elicited by the electrical stimulation.

A signal representative of voltages in response to electrical stimulation as a function of time using a third combination of electrodes including multiple stimulating electrodes and multiple stimulating electrodes may have an amplitude and a duration of an initial peak that is decreased relative to the signals associated with the first and second combinations of electrodes. The signal may further include a noise floor of about 0.89 µV, a peak-to-peak noise of about 4.6 µV, and a signal-to-noise ratio of about 2.6. This signal may be indicative of stimulation artifacts associated with the implantable stimulator system that are less than the stimulation artifacts associated with the first and second combinations of electrodes.

A signal representative of voltages in response to electrical stimulation as a function of time using a fourth combination of electrodes including a contralateral recording configuration (e.g., where recording occurs at a first ear of a recipient and stimulation is provided at a second ear) may not include a noticeable initial peak. Moreover, the signal may include a noise floor of about 0.87 µV, a peak-to-peak noise of about 3.9 µV, and a signal-to-noise ratio of about 2.2. This signal may be indicative of stimulation artifacts associated with the implantable stimulator system that are less than the stimulation artifacts associated with the other combinations of electrodes.

Accordingly, the examples described above show that the stimulation artifacts associated with the implantable stimulator system are reduced by using multiple stimulating and recording electrodes. Moreover, the contralateral recording configuration resulted in little to no stimulation artifacts.

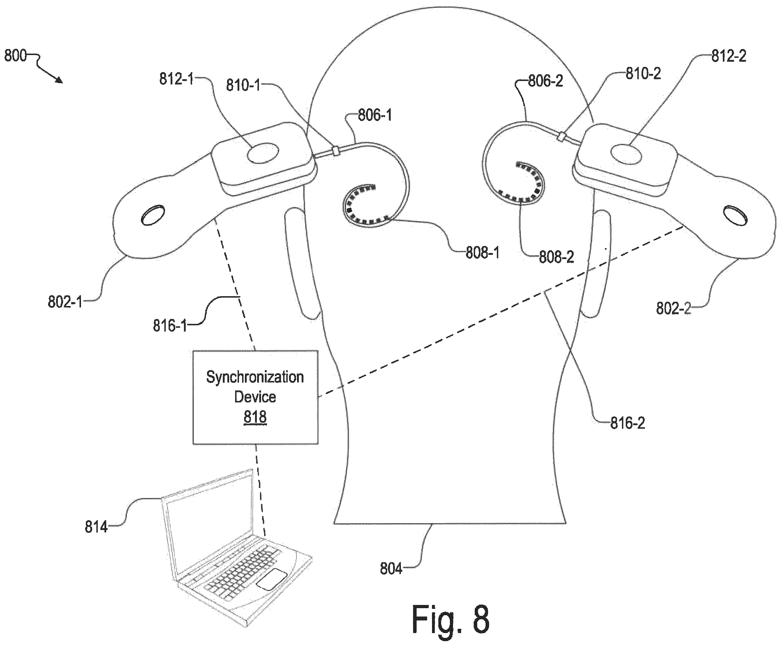

In some implementations, a contralateral recording configuration may be implemented by an implantable stimulator system including bilateral cochlear implants. For example, FIG. 8 shows an illustrative configuration 800 of an implantable stimulator system in which cochlear implants 802 (e.g., cochlear implants 802-1 through 802-2) are provided at each ear of recipient 804. As shown, each cochlear implant 802 is depicted in relation to a head of recipient 804. While FIG. 8 shows cochlear implants 802 as being partially apart from the head of recipient 804 for illustrative purposes, it is understood that cochlear implants 802 are implanted within recipient 804. As shown in FIG. 8, cochlear implants 802 are each electrically coupled to an electrode lead 806 (e.g., electrode leads 806-1 through 806-2) that includes electrodes 808 (e.g., electrodes 808-1 through 808-2) on a distal end and a ring electrode 810 (e.g., ring electrodes 810-1 through 810-2) on a proximal end towards each cochlear implant 802. Each cochlear implant 802 further includes a case electrode 812 (e.g., case electrodes 812-1 through 812-2).

During use, electrical stimulation may be provided to a first side (e.g., the ear on the left side of the head) of the head using one or more electrodes 808 (e.g., first electrodes 808-1). Any suitable combination of one or more other electrodes 808 (e.g., second electrodes 808-2, ring electrode 810-2, and/or case electrode 812-2) on a second side (e.g., the right side) of the head may be used to continuously record one or more signals in response to the electrical stimulation. Cochlear implant 802 associated with recording the one or more signals may stream the data to a computing device 814 by way of a back telemetry channel 816 (e.g., channels 816-1 through 816-2). Computing device 814 is shown as a laptop computer in FIG. 8 but could be implemented by any other suitable computing device such as described herein. Additionally or alternatively, a synchronization device 818 may be used to synchronize one or more operations of the implantable stimulator system. For example, synchronization device 818 may be configured to synchronize the stimulation applied to elicit an evoked response and the recording of the one or more signals, though synchronization device 818 is optional and may be omitted in some implementations.

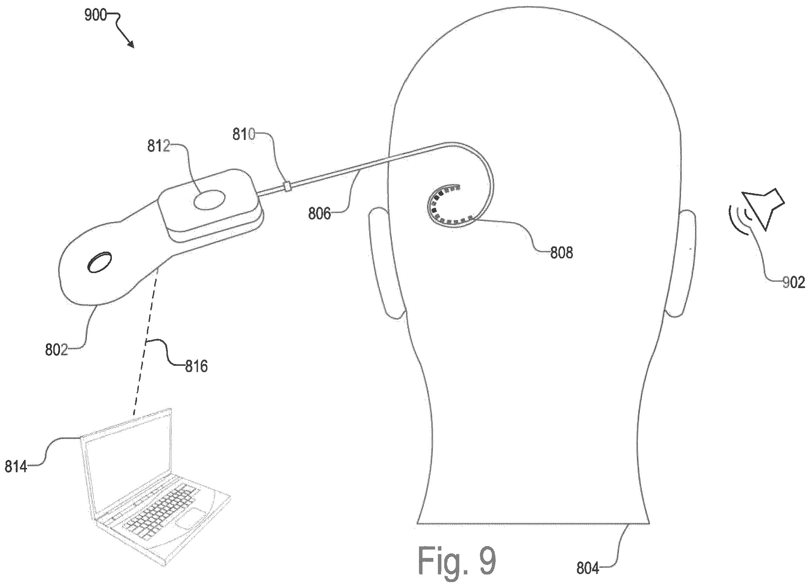

In some implementations, the stimulation provided by an implantable stimulator system may include a combination of electrical stimulation and acoustic stimulation. As an illustrative example, FIG. 9 shows an illustrative configuration 900 of an implantable stimulator system that is similar to configuration 800 shown in FIG. 8 except that an acoustic stimulation source 902 is provided in a vicinity of an ear of recipient 804. In the example shown in FIG. 9, acoustic stimulation source 902 may be configured to concurrently apply acoustic stimulation with electrical stimulation applied by one or more electrodes 808 of cochlear implant 802. The acoustic stimulation may be representative of an audio signal (e.g., an amplified version of the audio signal) configured to elicit an evoked response within the recipient. Acoustic stimulation source 902 may be implemented by a speaker, an output transducer, and/or a receiver of an ITE hearing device of hearing system. Any suitable combination of non-stimulated electrodes 808, ring electrode 810, and/or case electrode 812 may be used to continuously record one or more signals in response to the electrical stimulation and acoustic stimulation. Cochlear implant 802 may stream the one or more signals to computing device 814 by way of back telemetry channel 816. Still other suitable configurations of an implantable stimulator system may be used. For example, additional electrodes (not shown) separate from electrode lead 806 may be implanted within recipient 804 and electrically coupled to cochlear implant 802.

Although the preceding description is described in the context of a cochlear implant system, it is understood that concepts such as those described herein may be applied in other contexts with other types of implantable stimulator systems.

In some examples, a non-transitory computer-readable medium storing computer-readable instructions may be provided in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Exemplary non-volatile storage media include, but are not limited to, read-only memory, flash memory, a solid-state drive, a magnetic storage device (e.g., a hard disk, a floppy disk, magnetic tape, etc.), ferroelectric random-access memory (RAM), and an optical disc (e.g., a compact disc, a digital video disc, a Blu-ray disc, etc.). Exemplary volatile storage media include, but are not limited to, RAM (e.g., dynamic RAM).

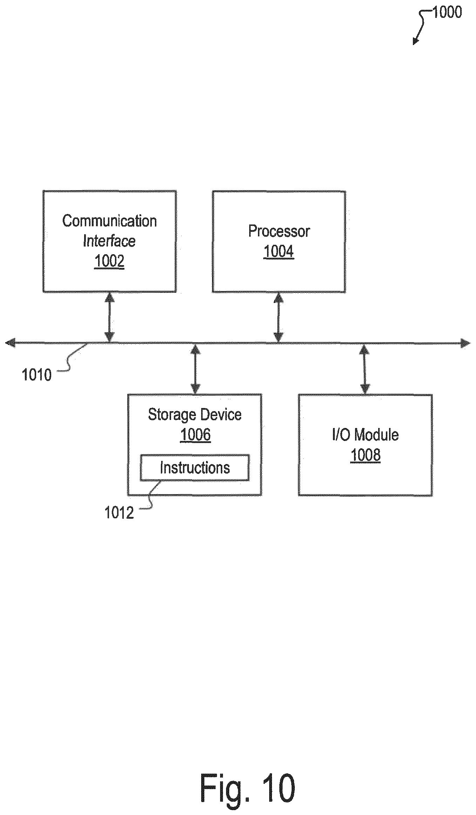

FIG. 10 illustrates an exemplary computing device 1000 that may be specifically configured to perform one or more of the processes described herein. As shown in FIG. 10, computing device 1000 may include a communication interface 1002, a processor 1004, a storage device 1006, and an input/output (I/O) module 1008 communicatively connected one to another via a communication infrastructure 1010. While an exemplary computing device 1000 is shown in FIG. 10, the components illustrated in FIG. 10 are not intended to be limiting. Additional or alternative components may be used in other embodiments. Components of computing device 1000 shown in FIG. 10 will now be described in additional detail.

Communication interface 1002 may be configured to communicate with one or more computing devices. Examples of communication interface 1002 include, without limitation, a wired network interface (such as a network interface card), a wireless network interface (such as a wireless network interface card), a modem, an audio/video connection, and any other suitable interface.

Processor 1004 generally represents any type or form of processing unit capable of processing data and/or interpreting, executing, and/or directing execution of one or more of the instructions, processes, and/or operations described herein. Processor 1004 may perform operations by executing computer-executable instructions 1012 (e.g., an application, software, code, and/or other executable data instance) stored in storage device 1006.

Storage device 1006 may include one or more data storage media, devices, or configurations and may employ any type, form, and combination of data storage media and/or device. For example, storage device 1006 may include, but is not limited to, any combination of the non-volatile media and/or volatile media described herein. Electronic data, including data described herein, may be temporarily and/or permanently stored in storage device 1006. For example, data representative of computer-executable instructions 1012 configured to direct processor 1004 to perform any of the operations described herein may be stored within storage device 1006. In some examples, data may be arranged in one or more databases residing within storage device 1006.

I/O module 1008 may include one or more I/O modules configured to receive user input and provide user output. One or more I/O modules may be used to receive input for a virtual experience. I/O module 1008 may include any hardware, firmware, software, or combination thereof supportive of input and output capabilities. For example, I/O module 1008 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1008 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1008 is configured to provide graphical data to a display for presentation to a user. The graphical data may be representative of one or more graphical user interfaces and/or any other graphical content as may serve a particular implementation. In certain embodiments, I/O module 1008 may be configured to provide a trigger signal associated with any of the operations described herein. For example, the trigger signal provided by I/O module 1008 may be used to facilitate detecting evoked responses during bilateral stimulation.

In some examples, any of the systems, computing devices, and/or other components described herein may be implemented by computing device 1000. For example, memory 110 may be implemented by storage device 1006, and processor 112 may be implemented by processor 1004.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A system comprising:
a memory storing instructions; and
a processor configured to execute the instructions to perform a process comprising:
measuring electrical statuses for each electrode included in a plurality of electrodes of an implantable stimulator that is implanted within a recipient;
designating, based on the electrical statuses, a first set of one or more electrodes included in the plurality of electrodes as stimulating electrodes to be used to deliver electrical stimulation to the recipient; and
designating, based on the electrical statuses, a second set of one or more electrodes included in the plurality of electrodes as recording electrodes to be used to record one or more signals representative of one or more evoked responses that occur within the recipient in response to the electrical stimulation.

2. The system of claim 1, wherein the plurality of electrodes includes at least one electrode located on an electrode lead connected to a cochlear implant and at least partially inserted within a cochlea of the recipient.

3. The system of claim 1, wherein the measuring the electrical statuses includes measuring impedances for each electrode included in the plurality of electrodes, the measuring the impedances comprising:
sequentially applying current by way of each electrode included in the plurality of electrodes;
measuring voltages associated with the current at each electrode; and
determining, based on the voltages, an impedance value representative of an impedance for each electrode.

4. The system of claim 3, wherein the measuring the voltages includes measuring voltages between each stimulated electrode and one or more return electrodes, the one or more return electrodes including one or more of a designated ground electrode, a case of the implantable stimulator, or one or more non-stimulated electrodes included in the plurality of electrodes.

5. The system of claim 3, wherein the designating the first set of one or more electrodes and the second set of one or more electrodes is based on determining one or more minimum impedance values.

6. The system of claim 1, wherein the designating the first set of one or more electrodes and the second set of one or more electrodes is further based on the second set of one or more electrodes being spaced a minimum distance away from the first set of one or more electrodes.

7. The system of claim 1, wherein the measuring the electrical statuses includes measuring stimulation artifacts for combinations of electrodes included in the plurality of electrodes, wherein the measuring the stimulation artifacts includes directing the combinations of electrodes to sequentially apply electrical stimulation and record one or more signals representative of stimulation artifacts in response to the electrical stimulation.

8. The system of claim 7, wherein the measuring the stimulation artifacts includes applying the electrical stimulation below a stimulation threshold at which the one or more evoked responses are evoked, or
wherein the measuring the stimulation artifacts includes applying the electrical stimulation with varying stimulation parameters, or
wherein the measuring the stimulation artifacts includes applying the electrical stimulation using asymmetric pulses.

9. The system of claim 7, wherein the designating the first set of one or more electrodes and the second set of one or more electrodes is based on reducing the stimulation artifacts.

10. The system of claim 1, wherein the first set of one or more electrodes is positioned at a first ear of the recipient and the second set of one or more electrodes is positioned at a second ear of the recipient.

11. The system of claim 1, wherein the second set of one or more electrodes are configured to continuously record the one or more signals during an acquisition window.

12. The system of claim 11, wherein the acquisition window is between 0 seconds and 2 seconds from applying the electrical stimulation.

13. The system of claim 1, wherein the one or more evoked responses include one or more auditory evoked potentials from auditory cortical areas.

14. The system of claim 1, wherein the process further comprises processing the one or more signals to determine one or more properties of the one or more evoked responses.

15. A cochlear implant system comprising:
a cochlear implant including a plurality of electrodes positioned on an electrode lead at least partially within a cochlea of a recipient; and
a processing unit communicatively coupled to the cochlear implant and configured to:
measure electrical statuses for each electrode included in the plurality of electrodes;
designate, based on the electrical statuses, a first set of one or more electrodes included in the plurality of electrodes as stimulating electrodes to be used to deliver electrical stimulation to the recipient; and
designate, based on the electrical statuses, a second set of one or more electrodes included in the plurality of electrodes as recording electrodes to be used to record one or more signals representative of one or more evoked responses that occur within the recipient in response to the electrical stimulation.
